# EUROPÄISCHE PATENTSCHRIFT

(11) **EP 4 580 526 B1**
(45) Veröffentlichungstag und Bekanntmachung des Hinweises auf die Patenterteilung: **18.11.2026**
(21) Anmeldenummer: 23847741.8
(22) Anmeldetag: 07.12.2023
(51) Int. Cl.: A61B 17/29, B33Y 80/00, A61B 17/32, A61B 34/30

(54) **WERKZEUG ZUR ANWENDUNG ALS MIKROMANIPULATOR UND VERFAHREN ZUR HERSTELLUNG DES WERKZEUGES**
TOOL FOR USE AS A MICROMANIPULATOR AND METHOD FOR PRODUCING THE TOOL
OUTIL DESTINÉ À ÊTRE UTILISÉ EN TANT QUE MICROMANIPULATEUR ET PROCÉDÉ DE PRODUCTION DE L'OUTIL

(30) Priorität: 08.12.2022 DE 202022106858 U; 06.12.2023 DE 102023134213
(43) Veröffentlichungstag der Anmeldung: 09.07.2025
(73) Patentinhaber: 3D Microprint GmbH, 09126 Chemnitz (DE)
(72) Erfinder: BLAU, Michael, 09114 Chemnitz (DE); GÖBNER, Joachim, 07616 Bürgel (DE); STARKE, Thomas, 09130 Chemnitz (DE)
(74) Vertreter: Rumrich, Gabriele
(86) Internationale Anmeldenummer: PCT/DE2023/100947
(87) Internationale Veröffentlichungsnummer: WO 2024/120583

(56) Entgegenhaltungen:
- WO-A2-2013/025831
- CN-A- 110 051 409
- US-A1- 2010 023 050
- US-A1- 2022 346 763

## Beschreibung

Die vorliegende Erfindung betrifft ein Werkzeug zur Anwendung als Mikromanipulator und ein Verfahren zur Herstellung des Werkzeuges nach dem Oberbegriff des 1. und 9. Patentanspruchs.

Grundsätzlich sind Mikromanipulatoren beziehungsweise Greifer, die als Mikromanipulatoren verwendet werden, eine Baugruppe aus mehreren Einzelteilen, welche aus fertigungstechnischer Sicht im 3D-Druck wie mehrere ineinander verschachtelte Bauteile behandelt werden. Beweglichkeit und Funktionalität derartiger Greifer werden üblicherweise durch Schweißen, Kleben, Löten, Pressen und anderen Fügeverfahren erzeugt. Die Fertigung und Montage bedarf mehrerer Einzelschritte und ist aufwändig und fehleranfällig. Auch sind die Verbindungspunkte der einzelnen Bauteile anfällig für Verschmutzungen und/oder Verschleiß.

Beispielsweise offenbart die DE 101 36 581 A1 ein Verfahren zur Messung und/oder Regelung der Greifkraft eines Mikromanipulators, mit dem Gegenstände mit mikroskopischen Abmessungen, insbesondere im Bereich zwischen einem Mikrometer und einem Millimeter, greifbar sind, wobei der Mikromanipulator zwei gegeneinander verschiebliche Klemmbacken aufweist, zwischen die ein Gegenstand unter Aufbringung einer Klemmkraft klemmbar ist, wobei eine der Klemmbacken 2 als Teil einer Federzunge 3 ausgebildet ist, die in Eigenschwingung versetzt wird, wobei die Änderung der Schwingung während der Berührung des Gegenstandes 1 durch die Federzunge 3 beobachtet wird, wobei aus der Änderung eine die Klemmkraft charakterisierende Größe berechnet und wobei die Greifkraft anhand der charakterisierenden Größe gesteuert wird.

Die EP 2 120 818 A1 beschreibt ein Verfahren zur Herstellung einer chirurgischen Mikromanipulatorspitze, die eine proximale Montagebasis, mindestens einen ersten Krümmer und einen distalen Manipulatorfinger umfasst, der über den ersten Krümmer mit der Montagebasis verbunden ist und am freien Ende der Spitze endet. Darüber hinaus umfasst die Befestigungsbasis mindestens ein distales Segment. Der erste Krümmer und der Manipulatorfinger bilden zusammen einen Spitzenabschnitt, und die Erfindung ist dadurch gekennzeichnet, dass der Spitzenabschnitt durch Anspitzen gleichmäßig verjüngt ist.

Aus der Druckschrift DE 10 2008 051 866 B3 ist ein Funktionsmodul für ein chirurgisches Element bekannt, welches ein Zangenelement aufweist. Eine klappbare Klaue ist an einer starren Klaue angelenkt, die fest mit einer Hülse verbunden ist, die ein Außengewinde trägt, das in ein korrespondierendes Innengewinde des Schaftes einschraubbar ist. Die Klingenbetätigungsstange mündet distal in einem Führungskolben, der einen radialen Führungsvorsprung aufweist, der in einem Axialschlitz der Führungshülse geführt ist. Der Führungskolben weist eine mit einer Ausnehmung auf, in die der Klingenschaft einer Schneidklinge einsteckbar ist.

In der Druckschrift DE 10 2006 050 469 A1 wird ein Greifer für Rohrförmige Werkstücke mit einer Antriebeinheit und zwei gegeneinander verschwenkbaren Greifarmen beschrieben. Damit soll ein zuverlässiges Greifen von stark gekrümmten, insbesondere rohrförmigen Werkstücken gewährleistet werden, wobei die Greifarme mit Druckstücken zum Fixieren des Werkstückes ausgerüstet sind. Die Druckstücke bestehen aus einem hitzebeständigen insbesondere keramischen Material. Diese Lösung ist nicht für endoskopische Anwendungen vorgesehen.

Ein Werkzeugkopf für endoskopische Anwendungen ist aus der Druckschrift DE 20 2004019 910 U1 bekannt. Bei diesem wird die Werkzeugaufnahme durch einen umgeformten Blechabschnitt gebildet.

Gemäß der Druckschrift DE 20 2005 005 406 U1 weist ein medizinisches Instrument mit einem distalen Endbereich relativ zueinander bewegliche, um einen Achszapfen verschwenkbare Maulteile auf, wobei der Kopf des Achszapfens als Vieleck ausgebildet ist. Der Achszapfen weist eine Buchse und einen in diese einführbaren Stift auf. Stift und Buchse sind miteinander verschraubt oder verpresst. Weiterhin kann zwischen diesen eine Klemmmutter angeordnet sein.

Eine Gewebezange wird in US 2020/0107873 offenbart, bei der wie bei den vorgenannten Lösungen, eine Vielzahl von Einzelteilen montiert werden müssen.

Bei allen gängigen Mikromanipulatoren müssen somit mehrere einzelne Bauteile gefertigt und zusammen montiert werden, was einen hohen Aufwand bedeutet und Fehlerquellen in sich birgt, beispielsweise hinsichtlich einzuhaltender Toleranzen. Weiterhin besteht bei der Verwendung unterschiedlicher Materialien die Gefahr, dass Korrosion auftritt.

In der Druckschrift US 2022/346763 A1 wird eine endoskopische Zange beschrieben, welche einen Betätigungsteil, ein Gehäuse und zwei bewegliche Zangen aufweist, welche in dem Gehäuse aufgenommen sind und durch den Betätigungsteil geöffnet und geschlossen werden können. Dieses medizinische Gerät kann beispielsweise durch ein additives Fertigungsverfahren hergestellt werden. Dazu können Materialschichten mit einer Dicke von 5 bis 50 Mikrometern auf einer Bauplattform abgelegt und die Schichten selektiv gesintert werden. Dafür werden Stützstrukturen mit erzeugt, die später entfernt werden müssen. Formungsprozesses stützen und nach der Formung entfernt werden. Die Trennung der Stützstrukturen kann auch dazu beitragen, Komponenten wie die Zangen von dem Gehäuse zu trennen, damit die Komponenten relativ zueinander beweglich sind. Dies bedeutet einen erhöhten fertigungstechnischen Aufbau und die Verwendung von zusätzlichem Pulver für die Stützstrukturen. Das Betätigungsteil weist einen Ringabschnitt auf, der in einer Kammer des Gehäuses verschiebbar zwischen zwei Positionen bewegbar ist, einer vorderen Position, bei welcher die Zangen geöffnet sind und einer hinteren Position, bei welcher die Zangen geschlossen sind. Das Betätigungsteil weist weiterhin in Richtung der Zangen ein Steuerteil auf. Zum Öffnen einer Zange gleitet das Steuerteil mit einer Fläche auf einer angrenzenden Fläche der jeweiligen Zange entlang. Die dabei entstehende Reibung kann zu unerwünschten Verschleißerscheinungen führen.

Die Druckschrift US 2010 023 050 A1 beschreibt eine chirurgische Greifzange mit zwei Greifbacken. Jede Greifbacke weist einen relativ zum Grundkörper ortsfesten Drehzapfen und einen Hebelarm auf und die Hebelarme sind über mindestens ein Schubelement gelenkig miteinander verbunden. Die Greifbacken, das die betätigende Schubelement und ein gabelförmiges Gehäuse werden aus Einzelteilen gefertigt und aufwendig montiert. Über einen Führungsstift, der durch das gabelförmige Gehäuse und die Greifbacken reicht, werden diese Teile miteinander verbunden. Wie der Führungsstift gesichert ist, wird nicht offenbart. Die Herstellung in einem Verfahrensablauf durch Mikrolasersintern wird bei dieser konstruktiv aufwendigen Greifzange nicht in Erwägung gezogen.

Eine weitere Greifzange für chirurgische Anwendungen wird in der Druckschrift WO 2013/025831 A1 beschrieben. Diese Greifzange kann aus einem einzigen Materialstück, beispielsweise Kunststoff oder einer Metalllegierung, geschnitten oder geformt werden. Beispielsweise kann die einteilige Backenstruktur 110 durch Schneiden und Fräsen von Polypropylen-Kunststoff oder einer Metalllegierung hergestellt werden oder durch Verwendung eines elektroerosiven Bearbeitungsverfahrens (EDM) bzw. durch Spritzgießen. Ein additives Verfahren wird nicht in Erwägung gezogen. Durch die einteilige konstruktiv aufwendige Ausbildung ist keine aufwendige Montage erforderlich. Allerdings ist durch die einteilige Ausführung und die dafür erforderliche Verwendung von Festkörperscharnieren der Bewegungsradius begrenzt.

Der Erfindung liegt die Aufgabe zugrunde, eine Alternative zu konventionell gefertigten Mikromanipulatoren zu schaffen, welche in einem einzigen Arbeitsschritt gefertigt werden können, wodurch keine Montagearbeiten mehr erforderlich sind und welche bei ähnlichen Öffnungswinkeln und Einbauräumen einen einfacheren und funktionssicheren Aufbau aufweisen.

Die Aufgabe wird durch die Merkmale des 1. und 9. Anspruches gelöst. Weitere zweckmäßige Ausgestaltungen der Erfindung sind Gegenstand der abhängigen Ansprüche.

Auch offenbart ist ein Werkzeug zur Anwendung als Mikromanipulator, aufweisend ein entlang einer Längsachse ausgerichtetes Gestell, welches als Hohlkörper um einen Innenraum herum angeordnet ist. Angeordnet an einer ersten Stirnseite weist das erfindungsgemäße Werkzeug wenigstens ein Manipulatorelement auf. Angeordnet an einer der ersten Stirnseite gegenüberliegenden zweiten Stirnseite weist das erfindungsgemäße Werkzeug wenigstens ein Betätigungselement auf, welches in dem Innenraum des Gestells gleitgelagert und axial beweglich ist. Erfindungsgemäß sind das Gestell, das wenigstens eine Betätigungselement und das wenigstens eine Manipulatorelement formschlüssig und beweglich miteinander verbunden und das Werkzeug komplett durch additive Fertigung hergestellt, einschließlich aller erforderlicher Spalte zwischen den beweglich miteinander verbunden Teilen wobei die Spalte maximal 25µm betragen und,
- wobei eine erste Greifbacke mit dem Gestell und dem wenigstens einen Betätigungselement beweglich verbunden ist und eine zweite Greifbacke mit dem Gestell starr verbunden ist
   oder
- wobei beide Greifbacken mit dem Gestell und dem wenigstens einen Betätigungselement beweglich verbunden sind
   und wobei
- die mit dem Gestell und dem wenigstens einen Betätigungselement beweglich verbundene/n Greifbacke/n drehbar um eine Achse eines zweiten Lagerstiftes angeordnet ist/sind, welcher quer zur Längsachse des Gestells angeordnet ist
- die beweglich angeordnete Greifbacke in einem Gelenkbereich eine erste/zweite Aufnahme in Form einer Ausnehmung aufweist, in welche ein am Betätigungselement angeordneter Betätigungsstift greift,
- in dem Gelenkbereich ein am Gestell angeordneter Führungsstift in ein Langloch eines Greifbackens greift
   und wobei
- das Gestell, das Manipulatorelement, das Betätigungselement und die Betätigungsstifte sowie die Lagerstifte und die Führungsstifte aus dem gleichen Material bestehen, wobei die Lagerstifte und Führungsstifte mit dem Gestell einteilig ausgebildet sind und die Betätigungsstifte mit dem Betätigungselement einteilig ausgebildet sind.

Das erfindungsgemäße Verfahren zur Herstellung eines Werkzeuges zur Anwendung als Mikromanipulator gemäß Anspruch 9, ist dadurch gekennzeichnet, dass das Werkzeug mit den Spalten zwischen den beweglichen Teilen komplett durch additive Fertigung mittels Mikrolasersintern in einem Bearbeitungsvorgang gefertigt wird, wobei ausgehend von einer Längsseite des Werkzeuges, dieses Schicht für Schicht gebaut wird, wobei jede Schicht aus einem metallischen oder keramischen Pulver mit einer Partikelgröße kleiner 20µm aufgetragen und anschließen mittels Laser geschmolzen wird und sich dann verfestigt, wobei das Pulver in Bereichen der Spalten zwischen den zu erzeugenden Teilen, die zueinander beweglich sein sollen, nicht geschmolzen wird und dass während des Mikrolasersinterns das Gestell, das Manipulatorelement und das Betätigungselement Schicht für Schicht gebaut werden, wobei die Spalte zwischen diesen maximal 25 µm betragen und dass eine erste Schicht aus einem Pulver auf einer Bauplattform aufgebracht wird und das durch Mikrolasersintern fertig hergestellte Werkzeug anschließend durch Drahterodieren oder ein anderes Trennverfahren von der Bauplattform getrennt wird.

Der Mikromanipulator ist insbesondere für medizinische Anwendungen vorgesehen und soll bevorzugt für Minimal-invasive Eingriffe (minimalinvasive Chirurgie (MIC)) eingesetzt werden.

Das Gestell kann zylinderförmig oder in seiner Querschnittsfläche eckig ausgebildet sein.

Das Manipulatorelement ist funktionell mit dem Betätigungselement verbunden und wird durch dieses bewegt.

Dabei sind das Gestell, das wenigstens eine Manipulatorelement und das wenigstens eine Betätigungselement einschließlich der zwischen diesen erforderlichen Spalten aus ein und demselben Material additiv gefertigt.

Dabei kann das Werkzeug aus metallischem Werkstoff oder Keramik oder deren Kombinationen bestehen und durch Mikro Laser Sintern hergestellt sein. Bevorzugte Metalle sind rostfreier Stahl, Chirurgenstahl, Titan oder andere in der Medizintechnik übliche Metalle. Es ist ebenfalls denkbar, dass das Werkzeug aus einem Kunststoff gefertigt ist.

Bevorzugt weist das Manipulatorelement wenigstens zwei Greifbacken auf. Die wenigstens zwei Greifbacken sind dabei derart angeordnet, dass sie einen geschlossenen Zustand und einen geöffneten Zustand einnehmen können, wobei sie im geschlossenen Zustand mit jeweils einer Auflagefläche aneinander aufliegen. Zwischen den Auflageflächen können Objekte gegriffen werden.

In einer bevorzugten Ausführungsform ist eine erste Greifbacke mit dem Gestell und dem Betätigungselement beweglich verbunden, während eine zweite Greifbacke mit dem Gestell starr verbunden ist. Eine derartige Ausführungsform stellt eine einseitig öffnende Variante dar.

Bei der einseitig öffnenden Variante kann es ebenso vorgesehen sein, dass die zweite Greifbacke in den Volumenkörper des Gestells integriert ist, also einteilig mit dem Gestell ausgebildet ist.

In einer weiteren bevorzugten Ausführungsform sind beide Greifbacken mit dem Gestell und dem Betätigungselement beweglich verbunden. Eine derartige Ausführungsform stellt eine beidseitig öffnende Variante dar.

Dabei kann vorgesehen sein, dass jeweils eine Greifbacke in einem Verbindungsbereich eine erste Aussparung aufweist, in welche ein erster Vorsprung und/oder ein erster Stift des Gestells greift. Die erste Aussparung ist dabei zentral im Verbindungsbereich der jeweiligen Greifbacke angeordnet. Es kann weiterhin vorgesehen sein, dass jeweils eine Greifbacke, insbesondere eine Greifbacke, welche beweglich mit dem Gestell und/oder dem Betätigungselement verbunden ist, eine zweite Aussparung aufweist, in welche ein zweiter Vorsprung und/oder ein zweiter Stift des Gestells greift, sowie, dass jeweils eine Greifbacke eine dritte Aussparung aufweist, in welche ein Vorsprung und/oder ein Stift des Betätigungselementes greift (Betätigungsstift). Die zweite und die dritte Aussparung sind dabei jeweils im Randbereich des Verbindungsbereiches angeordnet. Dabei wird bei einer Bewegung des Betätigungselementes in axialer Richtung eine Kraft auf die jeweils mit dem Betätigungselement in Wirkverbindung stehende/n Greifbacke/n ausgeübt und die jeweilige/n Greifbacke/n bewegt.

Vorteilhafterweise sind die mit dem Gestell und dem Betätigungselement beweglich verbundene/n Greifbacke/n drehbar um eine Achse angeordnet.

Es ist denkbar, dass auf die erste Aussparung und den ersten Stift verzichtet werden kann.

Die beiden Greifbacken können in einer Ebene oder leicht versetzt zueinander angeordnet sein, wobei bei einer versetzten Anordnung zumindest gewährleistet ist, dass die Auflageflächen der Greifbacken im geschlossenen Zustand aufeinander aufliegen.

Es kann weiterhin vorgesehen sein, dass das Werkzeug wenigstens eine Lagesicherung zur axialen Fixierung des wenigstens einen Manipulatorelementes aufweist. Eine solche Lagesicherung kann beispielsweise in der Art eines beweglichen und/oder elastisch federnden und/oder starren Riegels beziehungsweise Vorsprunges ausgebildet sein.

Vorteilhafterweise wird durch die Lagesicherung gewährleistet, dass das wenigstens eine Manipulatorelement sich nicht von dem Stift löst, über welchen es mit dem Gestell und/oder dem Betätigungselement verbunden ist.

Das Manipulatorelement kann weiterhin bevorzugt als Scherenmechanismus oder als Spreizer ausgebildet sein. Auch der Scherenmechanismus oder der der Spreizer sind in diesem Fall beweglich mit dem Betätigungselement verbunden.

Vorzugsweise weist das Betätigungselement an seiner frei liegenden von dem Gestell abgewandten Stirnseite wenigstens eine Öffnung für eine Betätigung auf, z.B. für einen Seilzug. Über eine angebrachte Seilzugbetätigung kann das Betätigungselement in eine axiale Bewegung versetzt werden.

Es kann vorgesehen sein, dass das erfindungsgemäße Werkzeug als Roboter-Endeffektor ausgebildet ist.

Alternativ kann das erfindungsgemäße Werkzeug hydraulisch oder pneumatisch betrieben werden.

Vorzugsweise ist das erfindungsgemäße Werkzeug geeignet zur Anwendung in mikroinvasiven Untersuchungen und Operationen. Weitere denkbare Anwendungsgebiete sind beispielsweise in der Labortechnik und Weltraumforschung zu sehen.

Bevorzugt wird das erfindungsgemäße Werkzeug additiv durch Mikro Laser Sintern hergestellt.

Dabei können das Gestell, das Manipulatorelement und das Betätigungselement aus dem gleichen Material bestehen.

Verfahrensgemäß erfolgt die Herstellung des erfindungsgemäßen Werkzeuges zur Anwendung als Mikromanipulator dadurch, dass das Werkzeug mit all seinen Teilen und mit den Spalten zwischen den zueinander beweglichen Teilen komplett durch additive Fertigung in einem Bearbeitungsvorgang gefertigt wird.

Bevorzugt erfolgt dies durch Mikrolasersintern.

Dabei wird ausgehend von einer Längsseite des Werkzeuges dieses Schicht für Schicht gebaut, wobei jede Schicht aus einem metallischen oder keramischen Pulver mit einer Partikelgröße kleiner 20µm aufgetragen und anschließend mittels Laser geschmolzen wird und sich dann verfestigt, wobei das Pulver in Bereichen der Spalten zwischen den zu erzeugenden Teilen, die zueinander beweglich sein sollen, nicht geschmolzen wird.

Während des Mikrolasersinterns werden das Gestell, das Manipulatorelement und das Betätigungselement Schicht für Schicht gebaut, wobei die Spalte zwischen diesen maximal 25 µm betragen.

Vorteilhafter Weise wird zuerst eine erste Schicht aus einem Pulver auf einer Bauplattform aufgebracht, das Werkzeug durch Mikrolasersintern hergestellt/gebaut und das durch Mikrolasersintern fertig hergestellte Werkzeug anschließend durch Drahterodieren oder ein anderes Trennverfahren von der Bauplattform getrennt wird.

Verfahrensgemäß wird somit das Werkzeug in Form eines Mikromanipulators mit all seinen Teilen und mit den Spalten zwischen den zueinander beweglichen Teilen erstmalig komplett durch additive Fertigung in einem Bearbeitungsvorgang gefertigt.

Vorteilhafterweise ist das erfindungsgemäße Werkzeug als eine funktionale Baugruppe aufgrund der Möglichkeiten der additiven Herstellung fertigungstechnisch als ein einziges Bauteil ausgebildet, welches aus mehreren zueinander beweglichen Funktionsgruppen besteht. Somit entfallen mehrere Bearbeitungsschritte, die konventionell zur Fertigung einer Baugruppe notwendig sind: Fertigung mehrerer Einzelteile, Gewährleistung und Einhaltung von Fertigungstoleranzen der Einzelteile inklusive Qualitätssicherung, logistisches Zusammenführen der Einzelteile zur Montage, Montagearbeiten sowie Qualitätssicherungsarbeiten nach der Montage.

Grundsätzlich ist der Greifer eine Baugruppe aus mehreren Einzelteilen, welche aus fertigungstechnischer Sicht im 3D-Druck wie mehrere ineinander verschachtelte Bauteile behandelt wird. Der Greifer ist mittels Mikro Laser Sintern (MLS) aus Metall hergestellt. Die einzelnen Volumenkörper sind im fertigen Greifer ausschließlich über Formschluss und ohne einen Montage- oder Fügevorgang miteinander zu einem beweglichen Mechanismus verbunden. Dies unterscheidet das vorliegende Greiferdesign grundsätzlich von konventionell gefertigten Greifermechanismen aus Metall mit ähnlichen Öffnungswinkeln und Einbauräumen, deren Beweglichkeit und Funktionalität durch Schweißen, Kleben, Löten, Pressen und anderen Fügeverfahren erzeugt werden. Alle Einzelkörper und im Besonderen die Dreh- und Schiebeverbindungen sind nach den verfahrensspezifischen Designregeln für das MLS modelliert, sodass ein besonders geringes Spiel der beweglichen Elemente erzeugt wird. Dies und die gleichzeitig geringe Oberflächenrauheit der Bauteile ermöglichen eine spielarme Passung.

Alle innenliegenden Funktionsflächen der Dreh- und Schubgelenke und damit deren Funktion können ohne weitere Oberflächennachbearbeitung direkt nach dem Drucken und Trennen der Baugruppe von der Bauplattform genutzt werden.

Die funktionale Baugruppe in Form des Greifers wird aufgrund der Möglichkeiten des 3D-Druckes, bevorzugt des Mikrolasersintern, fertigungstechnisch als ein einziges Bauteil hergestellt, welches mehrere zueinander bewegliche Teile beinhaltet. Somit entfallen mehrere Bearbeitungsschritte, die konventionell zur Fertigung einer Baugruppe notwendig sind wie:
1. Fertigung mehrerer Einzelteile, unter Einhaltung der erforderlichen Toleranzen inklusive Qualitätssicherung,
2. Logistisches Zusammenführen der Einzelteile zur Montage,
3. Montagearbeiten,
4. Qualitätssicherungsarbeiten nach der Montage.

Die Erfindung wird nachfolgend anhand von Ausführungsbeispielen und zugehörigen Figuren näher erläutert, ohne dabei auf diese beschränkt zu sein. Es zeigen:
- Figur 1: eine Draufsicht des Werkzeuges;
- Figur 2: eine Seitansicht eines erfindungsgemäßen Werkzeuges im geschlossenen Zustand;
- Figur 3: den Längsschnitt A-A gemäß Figur 1 im geschlossenen Zustand;
- Figur 4: den Längsschnitt B-B gemäß Figur 1 im geschlossenen Zustand;
- Figur 5: eine Seitansicht eines erfindungsgemäßen Werkzeuges im geöffneten Zustand;
- Figur 6: den Längsschnitt A-A gemäß Figur 1 im geöffneten Zustand;
- Figur 7: den Längsschnitt B-B gemäß Figur 1 im geöffneten Zustand;
- Figur 8: eine perspektivische Ansicht eines erfindungsgemäßen Werkzeuges im teilweise geöffneten Zustand.

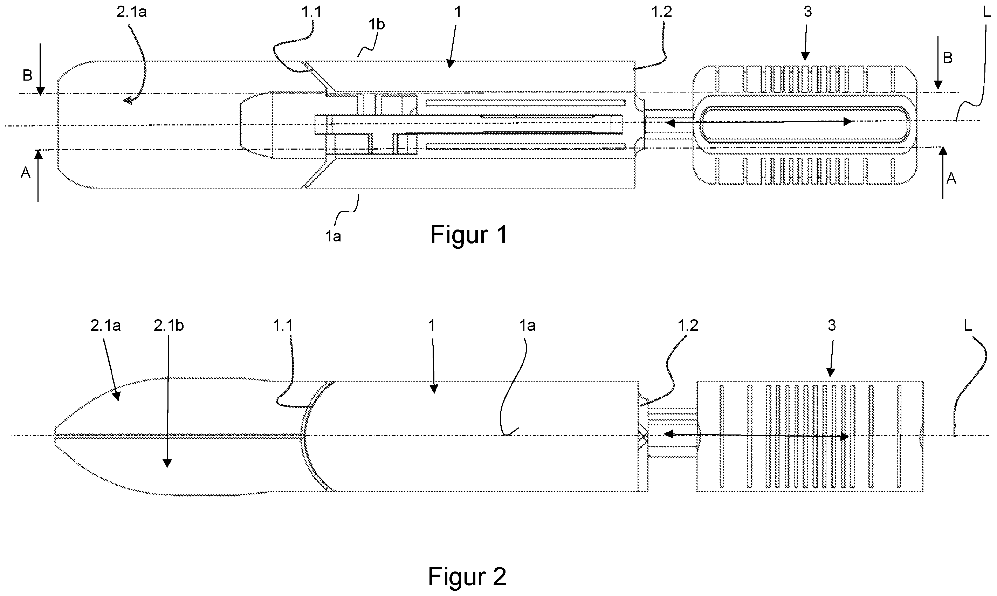

Die **Figuren 1 und** 2 zeigen ein erfindungsgemäßes Werkzeug in der Draufsicht und in der Seitenansicht im geschlossenen Zustand.

Das Werkzeug weist ein Gestell 1 auf, welches entlang einer Längsachse L ausgerichtet ist. Das Gestell 1 ist als Hohlkörper mit zwei sich gegenüberliegenden Öffnungen und einem Innenraum ausgebildet. Dabei weist das Gestell 1 eine erste Stirnseite 1.1 und eine der ersten Stirnseite 1.1 gegenüberliegende zweite Stirnseite 1.2 auf. An die erste Stirnseite 1.1 schließt sich ein Manipulatorelement 2 an, welches in der dargestellten Ausführungsform zwei Greifbacken 2.1a und 2.1b aufweist. Auf der Seite der zweiten Stirnseite 1.2 ist ein Betätigungselement 3 angeordnet, welches axial verschiebbar in dem Gestell 1 gelagert ist. Die axiale Beweglichkeit des Betätigungselementes 3 ist durch einen doppelseitigen Pfeil angedeutet. Das Betätigungselement 3 ist mit den Greifbacken 2.1a und 2.1b wirkverbunden.

Aus **Figur 1 und 2** ist ersichtlich, dass die Seitenflächen 1a und 1b des Gestells 1 glatt ausgeführt sind und die Lagerungselemente, welche das Schwenken der Greifbacken 2.1a und 2.1b realisieren, somit verdeckt angeordnet sind und somit innerhalb des Werkzeuges liegen.

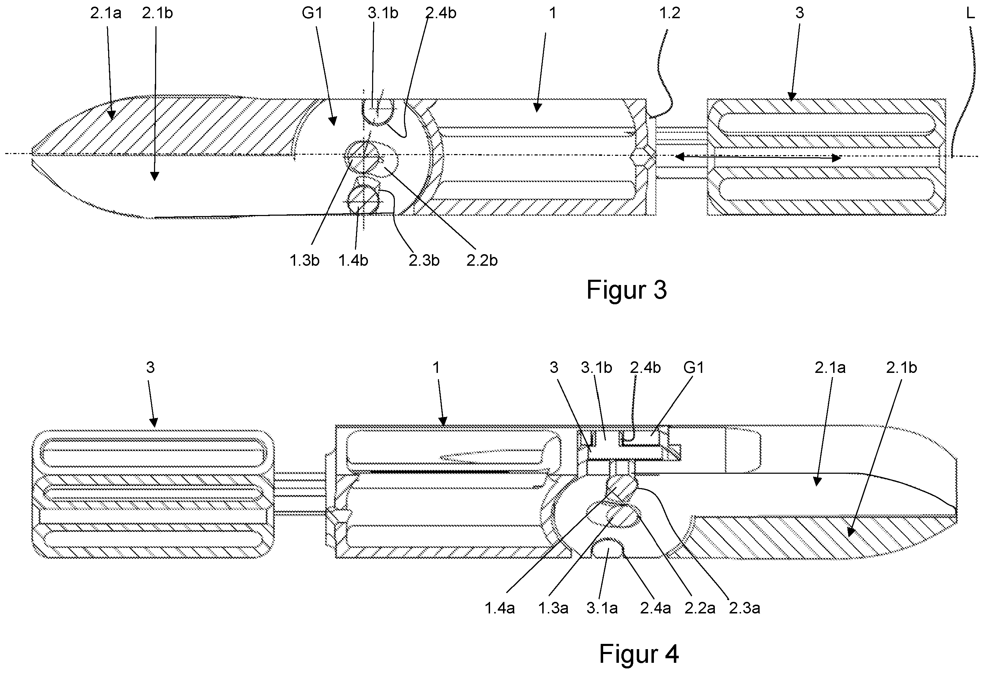

**Figur 3** zeigt den Längsschnitt A-A gemäß Figur 1 im geschlossenen Zustand aus Richtung der Längsseite 1a, wobei jedoch die hier untere Greifbacke 2.1b nicht mit einer Schraffur versehen ist. Es ist ersichtlich, dass die hier untere Greifbacke 2.1b mittels eines Gelenks G1, welches sich an die Greifbacke 2.1b einteilig anschließt, schwenkbar gelagert ist. Das Gelenk G1 ist ebenfalls nicht schraffiert. Das Gelenk G1 ist an seinem Außenumfang zumindest bereichsweise kreisförmig ausgebildet.

Der schwenkbar angeordnete hier untere erste Greifbacken 2.1b weist im ersten Gelenk G1 ein gebogenes erstes Langloch 2.2b auf, in welches ein am Gestell 1 quer zur Längsachse L und nach außen weisend angeordneter erster Führungsstift 1.3b greift.

Die beweglich angeordnete hier untere erste Greifbacke 2.1b weist weiterhin eine erste Lagerausnehmung 2.3b auf, in welche ein am Gestell 1 angeordneter erster Lagerstift 1.4b greift. Die beweglich angeordnete hier untere Greifbacke 2.1b weist weiterhin in ihrem ersten Gelenkbereich G1 erste Aufnahme 2.4b in Form einer Ausnehmung auf, in welche ein am Betätigungselement 3 angeordneter Betätigungsstift 3.1b greift.

Die erste Lagerausnehmung 2.3b und die erste Aufnahme 2.4b sind in einem Randbereich des Gelenkbereiches G1 angeordnet.

Der untere erste Greifbacken 2.1b ist um den ersten Lagerstift 1.4b drehbar/schwenkbar gelagert.

Der erste Führungsstift 1.3b und der erste Lagerstift 1.4b sind mit dem Gestell 1 einteilig ausgebildet.

Der erste Betätigungsstift 3.1b ist mit dem Betätigungselement 3 einteilig ausgebildet.

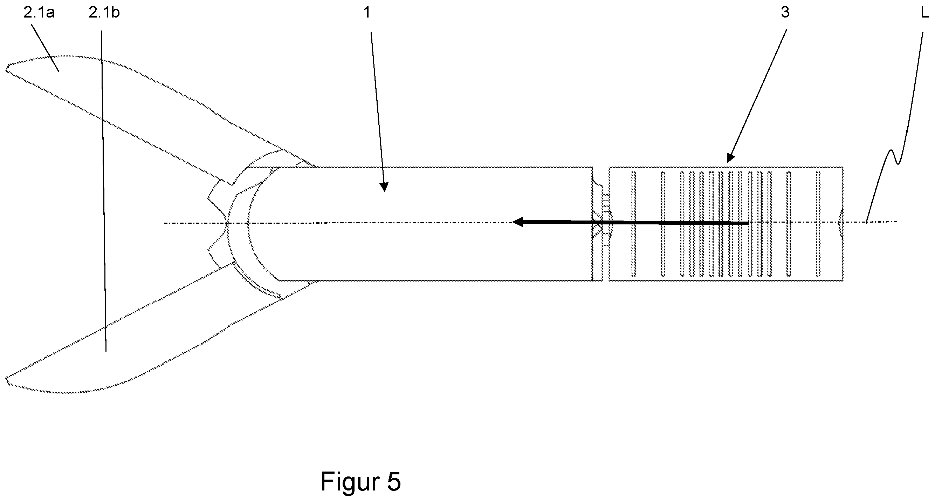

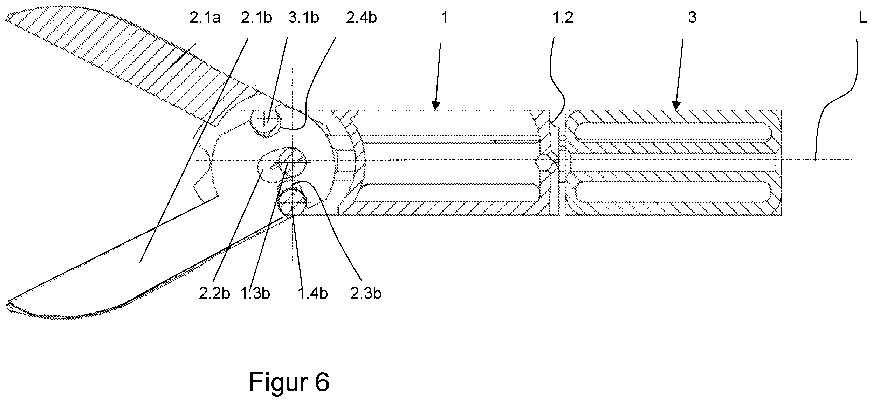

Wird das Betätigungselement 3 in Richtung zum Gestell 1 geschoben, so wird der am vorderen Ende des Betätigungselements 3 angeordnete erste Betätigungsstift 3.1b, welcher in die erste Aufnahme 2.4b des Gelenks G1 greift, ebenfalls axial hier nach links bewegt und nimmt dadurch das Gelenk G1 und die zugehörige Greifbacke 2.1b mit. Der Drehpunkt des ersten Gelenks G1 befindet sich dabei in der nicht bezeichneten Achse des ersten Lagerstiftes 1.4b. Somit schwenkt die hier untere Greifbacke 2.1b mit dem Gelenk G1 um den ersten Lagerstift 1.4b nach unten in die geöffnete Position (siehe Figuren 5 und 6).

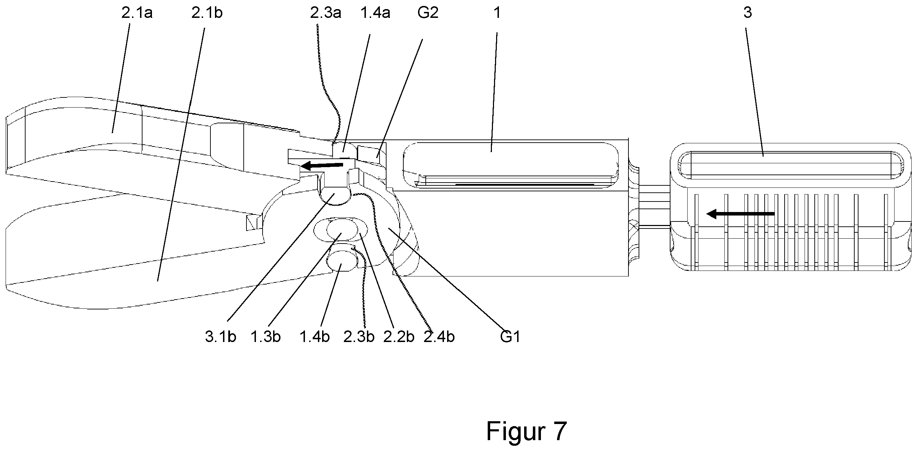

Um die obere zweite Greifbacke 2.1a nach oben zu Schwenken, befinden sich bei dieser in Richtung zum Gestell ein zweites Gelenk G1 (hier nicht ersichtlich) und
- ein zweiter Lagerstift 1.4a in einer zweiten Lagerausnehmung 2.3a des zweiten Gelenks G2,
- ein zweiter Führungsstift 1.3a in einem zweiten Langloch 2.2b des zweiten Gelenks G2 und
- ein zweiter Betätigungsstift 3.1a in einer zweiten Aufnahme 2.4a in Form einer Ausnehmung im zweiten Gelenk G2
im Bereich eines der oberen Greifbacke 2.1a zugeordneten zweiten Gelenks G2 (siehe Figur 4 und 7).

Der zweite Lagerstift 1.4a und der zweite Führungsstift 1.3a sind mit dem Gestell 1 einteilig ausgebildet.

Der zweite Betätigungsstift 3.1a ist mit dem Betätigungselement 3 einteilig ausgebildet.

Diese Ausbildung des zweiten Gelenks G2 ist in Bezug auf die Längsachse L um 180° gedreht. Daher befindet sich der Drehpunkt das Gelenks der hier oberen zweiten Greifbacke 2.1a oben und der Angriffspunkt des zweiten Betätigungsstiftes 3.1a unten, wodurch bei einer Bewegung des Betätigungselements 3 in Richtung zum Gestell 1 der obere zweite Greifbacken 2.1a nach oben geschwenkt wird.

**Figur 4** zeigt die geschlossene Position des Werkzeuges quer zur Längsachse L gedreht und von der anderen Seite aus im Teilschnitt. Dabei wurden das zweite Gelenk G2 und die obere zweite Greifbacke 2.1a nicht schraffiert.

Auf der hier dargestellten Seite des Werkzeuges sind die obere zweite Greifbacke 2.1a und das zweite Gelenk G2 einteilig ausgebildet und das Gestell 1 weist an seinem in Richtung zu den Greifbacken 2.1a und 2.1b weisenden Ende einen quer zur Längsachse L ausgerichteten zweiten Betätigungsstift 3.1a auf, mit dem das Gelenk G1 und die obere Greifbacke 2.1a betätigbar sind. Der zweite Betätigungsstift 3.1a greift dazu in eine Aussparung bzw. zweite Aufnahme 2.4a des zweiten Gelenks G2.

Das Gestell 1 weist einen zweiten Führungsstift 1.3a auf, der in einem zweiten Langloch 2.2a geführt wird und am Gestell 1 ist weiterhin ein zweiter Lagerstift 1.4a vorgesehen, der in einer Lagerausnehmung 2.3a geführt wird und um den das zweite Gelenk G2 bei Betätigung des Betätigungselements 3 schwenkbar ist.

Wird das Betätigungselement 3 in Richtung zum Gestell 1 entlang der Längsachse L bewegt, werden mittels der am Betätigungselement 3 angeordneten ersten und zweiten Betätigungsstifte Stifte 3.1a und 3.1b die Greifbacken 2.1a und 2.1b durch eine Schwenkbewegung der ersten und zweiten Gelenke G1, G2 um die ersten und zweiten Lagerstifte 1.4a und 1.4b geschwenkt und somit die Greifbacken 2.1a und 2.1b geöffnet.

**Figur 5** zeigt die Seitenansicht des Werkzeuges in geöffneter Position und Figur 6 den Längsschnitt durch die Ebene A-A gemäß Figur 1 ebenfalls in geöffneter Position.

Aus Figur 5 ist wie aus Figur 2 ersichtlich, dass die Betätigungsstifte, Lagerstifte und Führungsstifte sowie Aufnahmen, Langlöcher und Lagerausnehmungen und Gelenke von außen nicht zu sehen sind, da diese durch das außen liegende Material des Gestells verdeckt sind.

Es ist aus **Figur 6** erkennbar, dass das Betätigungselement 3 in Richtung zum Gestell 1 axial verschoben wurde und sich die Greifbacken 2.1a und 2.1b in einer geöffneten Position befinden.

Aus Figur 6 ist ersichtlich, dass durch den ersten Betätigungsstift 3.1b, der in die dritte erste Aufnahme 2.4b greift, der erste Gelenkbereich/das erste Gelenk G1 um den ersten Lagerstift 1.4b geschwenkt wurde. Das erste Gelenk G1 hat sich mit seinem ersten Langloch 2.2 relativ zum ersten Führungsstift 1.3b bewegt. Der erste Führungsstift 1.3b, der in dem ersten Langloch 2.2b gelagert ist, dient dabei zur Stabilisierung.

**Figur 7** zeigt das Werkzeug in dreidimensionaler Darstellung in teilweise geöffneter Position. Daraus ist ersichtlich, dass der hier obere zweite Greifbacken 2.1a in Richtung zum Gestell 1 einen zweite Gelenkbereich/ein zweites Gelenk G2 aufweist, dessen Drehpunkt um die nicht bezeichnete Achse des zweiten Lagerstiftes 1.4a liegt, der hier oben angeordnet ist. Der zweite Lagerstift 1.4a ist mit dem Gestell 1 einteilig ausgebildet und wird in einer zweiten Lagerausnehmung 2.3a des Gelenks G2 gelagert.

Die Führungsstifte 1.3a, 1.3b und die Lagerstifte 1.4a, 1.4b sind einteilig mit dem Gestell 1 und die Betätigungsstifte 3.1a, 3.1b einteilig mit dem Betätigungselement 3 ausgebildet.

Die beiden Greifbacken 2.1a und 2.1b besitzen aufeinander zuweisende nicht bezeichnete Greifflächen, die im geschlossenen Zustand aneinander anliegen. Die Greifflächen können mit einer Strukturierung versehen sein.

Vorzugsweise weist das Betätigungselement 3 an seiner frei liegenden von dem Gestell 1 abgewandten Stirnseite wenigstens eine nicht bezeichnete Öffnung für ein Zug- Druckelement 3.2 auf. Darüber können Zug- und Druckkräfte zum Öffnen und Schließen der Greifbacken 2.1a, 2.1b eingeleitet werden. Über eine entsprechende Betätigung kann somit das Betätigungselement 3 in eine axiale Bewegung versetzt und darüber die erforderliche Zug-Druck-Bewegung realisiert werden.

Da die Greifbacken 2.1a, 2.1b sich in einem geschlossenen Zustand befinden, sind die aufeinander aufliegenden Auflageflächen nicht zu erkennen.

Um somit aus einer geschlossenen Position der Greifbacken in eine geöffnete Position zu gelangen, wird das Betätigungselement in Richtung zum Gestell 1 geschoben. Dadurch bewegen sich beide Betätigungsstifte 3.1a, 3.1b in Richtung der Greifbacken 2.1a, 2.1b und die Gelenke G1, G2 werden um ihre Lagerstifte 1.4a, 1.4b gedreht.

Dadurch schwenkt mit dem ersten Gelenk G1 der in den Figuren unten liegende erste Greifbacken 2.1b um den unten angeordneten ersten Lagerstift 1.4b nach unten und der oben liegende zweite Greifbacken 2.1a mit dem zweiten Gelenk G2 um den oben angeordneten zweiten Lagerstift 1.4a nach oben.

Bevorzugt wird ein derartiger Greifer für medizinische Anwendungen aus Metall (z.B. Stahl, Titan) hergestellt. Es sind jedoch ggf. auch andere für das Lasersintern geeignete Materialien verwendbar, die bevorzugt auch für medizinische Anwendungen, wie minimal-invasive Chirurgische Anwendungen, eingesetzt werden können.

Alle übrigen Merkmale sind adäquat zu den Figuren 1 und 2 zu erkennen. Auf die entsprechende Stelle in der Beschreibung wird hiermit verwiesen.

Wie bereits vorgenannt ausgeführt, werden alle Teile es Werkzeuges durch ein Additives Fertigungsverfahren, bevorzugt durch Mikro Laser Sintern in einem Verfahrensablauf hergestellt.

Bekannterweise wird beim Mikro Laser Sintern das dafür vorgesehene Pulver aus dem erforderlichen Material (Metall, Keramik oder Kunststoff) mittels einer Vorrichtung auf eine Substratplatte aufgetragen. Ein Laserstrahl schmilzt die Bereiche des Pulvers, die sich verfestigen sollen. Danach wird die Substratplatte abgesenkt und eine weitere Schicht Pulver aufgetragen und mittels Laser verschmolzen. Durch diesen sich wiederholenden Ablauf wird Schicht für Schicht das komplette Werkzeug hergestellt.

Nach dem Herstellungsprozess und nach dem Abtrennen von einer Substratplatte durch Drahterodieren liegt das Werkzeug mit seinen zueinander beweglich gelagerten Teilen in einer einsatzbereiten Form vor, ohne irgendwelche Montagearbeiten.

Das für die Anwendung als Mikromanipulator vorgesehene Werkzeug in Form des Greifers weist im geschlossenen Zustand in einer kleinen Baugröße beispielsweise einen Durchmesser von 0,5mm bis 1,0 mm, bevorzugt 0,7 mm bis 0,9 mm und eine Gesamtlänge von beispielsweise 1,2 bis 1,7 mm, bevorzugt 1,4 mm bis 1,6 mm auf.

Bei einer größeren Bauform kann das für die Anwendung als Mikromanipulator vorgesehene Werkzeug im geschlossenen Zustand beispielsweise einen Durchmesser von 5,0mm bis 7,0 mm, bevorzugt 5,5 mm bis 6,5mm und eine Gesamtlänge von beispielsweise 12mm bis 20 mm, bevorzugt 13mm bis 15 mm aufweisen.

Es sind jedoch auch noch größere Abmessungen möglich, beispielsweise eine Gesamtlänge von 30mm bis 50 mm, bevorzugt 35mm bis 45 mm und auch entsprechend größere Durchmesser.

Die Abmessung des Werkzeuges in Form des Greifers/Mikromanipulators kann selbstverständlich den erforderlichen Anwendungsbedingungen und - Voraussetzungen für minimal-invasive Chirurgie angepasst werden.

Derartige Werkzeuge werden im Bereich der Medizintechnik auch als Zangen bezeichnet.

### Bezugszeichenliste

- 1: Gestell
- 1.1: erste Stirnseite des Gestells
- 1a: erste Seitenfläche des Gestells
- 1b: zweite Seitenfläche des Gestells
- 1.2: zweite Stirnseite des Gestells
- 1.3a: erster Führungsstift
- 1.3b: zweiter Führungsstift
- 1.4a: erster Lagerstift
- 1.4b: zweiter Lagerstift
- 2: Manipulatorelement
- 2.1a: erste Greifbacke/obere Greifbacke
- 2.1b: zweite Greifbacke /untere Greifbacke
- 2.2: erste Aussparung
- 2.2a: zweites Langloch
- 2.2b: erstes Langloch
- 2.3a: zweite Lagerausnehmung
- 2.3b: erste Lagerausnehmung
- 2.4a: zweite Aufnahme/Aussparung
- 2.4b: erste Aufnahme/Aussparung
- 2.5: Verbindungsbereich
- 3: Betätigungselement
- 3.1a: zweiter Betätigungsstift
- 3.1b: erster Betätigungsstift
- 3.2: Öffnungen für Zug- Druckelement
- G1: erstes Gelenk
- G2: zweites Gelenk
- L: Längsachse

## Patentansprüche

1. Werkzeug zur Anwendung als Mikromanipulator für medizinische Anwendungen in Form von minimal-invasiven Eingriffen (aus Beschreibung Seite 3, Zeilen 21-22) , aufweisend mehrere Teile in Form
- eines entlang einer Längsachse (L) ausgerichteten Gestells (1), welches als Hohlkörper ausgebildet ist, der an beiden Enden offen ist,
- wenigstens eines an einer ersten Stirnseite (1.1) des Gestells (1) angeordneten Manipulatorelementes (2), sowie
- wenigstens eines an einer der ersten Stirnseite (1.1) gegenüberliegenden zweiten Stirnseite (1.2) angeordneten Betätigungselementes (3), welches in dem Gestell (1) gleitgelagert und entlang der Längsachse (L) axial beweglich ist und mit dem Manipulatorelement in Wirkverbindung steht,
und dass die Teile in Form des Gestells (1), des wenigstens einen Betätigungselements (3) und des wenigstens einen Manipulatorelements (2) formschlüssig und beweglich miteinander verbunden sind und das Werkzeug komplett durch additive Fertigung erzeugt ist, einschließlich aller erforderlicher Spalte zwischen den beweglich miteinander verbunden Teilen, **dadurch gekennzeichnet,**
**dass** das wenigstens eine Manipulatorelement (2) wenigstens zwei Greifbacken (2.1a, 2.1b) aufweist
- wobei eine erste Greifbacke (2.1a) mit dem Gestell (1) und dem wenigstens einen Betätigungselement (3) beweglich verbunden ist und eine zweite Greifbacke (2.1b) mit dem Gestell (1) starr verbunden ist oder
- wobei die erste und die zweite Greifbacke (2.1a, 2.1b) mit dem Gestell (1) und dem wenigstens einen Betätigungselement (3) beweglich verbunden sind und am Betätigungselement (3) ein erster und zweiter Betätigungsstift (3.1b und 3.1a) ausgebildet sind, wobei
der schwenkbar angeordnete erste Greifbacken (2.1b) im ersten Gelenk (G1) ein gebogenes erstes Langloch (2.2b) aufweist, in welches ein am Gestell (1) quer zur Längsachse (L) und nach außen weisend angeordneter erster Führungsstift (1.3b) greift und der schwenkbar angeordnete zweite Greifbacken (2.1a) im zweiten Gelenk (G2) ein gebogenes zweites Langloch (2.2a) aufweist, in welches ein am Gestell (1) angeordneter zweiter Führungsstift (1.3a) greift,
die mit dem Gestell (1) und dem wenigstens einen Betätigungselement (3) beweglich verbundene erste und zweite Greifbacke (2.1a, 2.1b) drehbar um eine Achse eines ersten und zweiten Lagerstiftes (1.4a, 1.4b) angeordnet sind, welcher quer zur Längsachse (L) des Gestells (1) angeordnet ist, , sodass sich ein Drehpunkt des ersten Gelenks (G1) in einer Achse des ersten Lagerstiftes (1.4b) befindet und dass ein Drehpunkt des zweiten Gelenks (G2) in einer Achse des zweiten Lagerstiftes (1.4a) liegt,
- das Gestell (1), das Manipulatorelement (2), das Betätigungselement (3) und der erste und zweite Betätigungsstift (3.1a, 3.1b), der erste und zweite Lagerstift (1.4a, 1.4b) und der erste und zweite Führungsstift (1.3a, 1.3b) aus dem gleichen Material bestehen, wobei der erste und zweite Lagerstift (1.4a, 1.4b) und der erste und zweite Führungsstift (1.3a, 1.3b) mit dem Gestell (1) einteilig ausgebildet sind und der erste und zweite Betätigungsstift (3.1a, 3.1b) mit dem Betätigungselement (3) einteilig ausgebildet sind.

2. Werkzeug nach Anspruch 1, **dadurch gekennzeichnet, dass** das Werkzeug wenigstens eine Lagesicherung zur axialen Fixierung des wenigstens einen Manipulatorelementes (2) aufweist.

3. Werkzeug nach Anspruch 1, **dadurch gekennzeichnet, dass** das wenigstens eine Manipulatorelement (2) als Scherenmechanismus oder als Spreizer ausgebildet ist.

4. Werkzeug nach einem der Ansprüche 1 bis 3, **dadurch gekennzeichnet, dass** das wenigstens eine Betätigungselement (3) an seiner frei liegenden Stirnseite wenigstens eine Öffnung für eine Betätigung aufweist.

5. Werkzeug nach einem der Ansprüche 1 bis 4, **dadurch gekennzeichnet, dass** das Werkzeug als Roboter-Endeffektor ausgebildet ist.

6. Werkzeug nach einem der Ansprüche 1 bis 5, **dadurch gekennzeichnet, dass** das Werkzeug aus Metall oder einem keramischen Material besteht.

7. Werkzeug nach einem der Ansprüche 1 bis 6 zur Anwendung in mikroinvasiven Untersuchungen und Operationen.

8. Werkzeug nach einem der Ansprüche 1 bis 7, **dadurch gekennzeichnet, dass** es in einem einzigen Verfahrensablauf durch Mikro Laser Sintern hergestellt ist.

9. Verfahren zur Herstellung eines Werkzeuges zur Anwendung als Mikromanipulator nach Anspruch 1, **dadurch gekennzeichnet, dass** das Werkzeug mit den Spalten zwischen den beweglichen Teilen komplett durch additive Fertigung mittels Mikrolasersintern in einem Bearbeitungsvorgang gefertigt wird, wobei ausgehend von einer Längsseite des Werkzeuges, dieses Schicht für Schicht gebaut wird, wobei jede Schicht aus einem metallischen oder keramischen Pulver mit einer Partikelgröße kleiner 20µm aufgetragen und anschließen mittels Laser geschmolzen wird und sich dann verfestigt, wobei das Pulver in Bereichen der Spalten zwischen den zu erzeugenden Teilen, die zueinander beweglich sein sollen, nicht geschmolzen wird und dass während des Mikrolasersinterns das Gestell (1), das Manipulatorelement (2) und das Betätigungselement (3) Schicht für Schicht gebaut werden, wobei die Spalte zwischen diesen maximal 25 µm betragen und dass eine erste Schicht aus einem Pulver auf einer Bauplattform aufgebracht wird und das durch Mikrolasersintern fertig hergestellte Werkzeug anschließend durch Drahterodieren oder ein anderes Trennverfahren von der Bauplattform getrennt wird.

## Claims

1. Tool for use as a micromanipulator for medical applications in the form of minimally invasive procedures (from description page 3, lines 21-22), comprising multiple parts in the form of:
- a frame (1) aligned along a longitudinal axis (L), which is formed as a hollow body that is open at both ends,
- at least one manipulator element (2) arranged on a first end face (1.1) of the frame (1), and
- at least one actuating element (3) arranged on a second end face (1.2) opposite the first end face (1.1), which is slidably mounted in the frame (1) and axially movable along the longitudinal axis (L) and is in operative connection with the manipulator element,
and that the parts in the form of the frame (1), the at least one actuating element (3), and the at least one manipulator element (2) are form-fittingly and movably connected to one another, and the tool is completely produced by additive manufacturing, including all necessary gaps between the movably connected parts, **characterized in that** the at least one manipulator element (2) has at least two gripping jaws (2.1a, 2.1b),
- wherein a first gripping jaw (2.1a) is movably connected to the frame (1) and the at least one actuating element (3) and a second gripping jaw (2.1b) is rigidly connected to the frame (1), or
- wherein the first and the second gripping jaws (2.1a, 2.1b) are movably connected to the frame (1) and the at least one actuating element (3), and a first and second actuating pin (3.1b and 3.1a) are formed on the actuating element (3), wherein
the pivotably arranged first gripping jaw (2.1b) has a curved first elongated hole (2.2b) in the first joint (G1), into which a first guide pin (1.3b) arranged on the frame (1) transversely to the longitudinal axis (L) and pointing outwards engages, and the pivotably arranged second gripping jaw (2.1a) has a curved second elongated hole (2.2a) in the second joint (G2), into which a second guide pin (1.3a) arranged on the frame (1) engages,
the first and second gripping jaws (2.1a, 2.1b) movably connected to the frame (1) and the at least one actuating element (3) are arranged rotatably about an axis of a first and second bearing pin (1.4a, 1.4b), which is arranged transversely to the longitudinal axis (L) of the frame (1), such that a pivot point of the first joint (G1) is located in an axis of the first bearing pin (1.4b) and that a pivot point of the second joint (G2) lies in an axis of the second bearing pin (1.4a),
- the frame (1), the manipulator element (2), the actuating element (3), and the first and second actuating pin (3.1a, 3.1b), the first and second bearing pin (1.4a, 1.4b), and the first and second guide pin (1.3a, 1.3b) consist of the same material, wherein the first and second bearing pin (1.4a, 1.4b) and the first and second guide pin (1.3a, 1.3b) are formed in one piece with the frame (1) and the first and second actuating pin (3.1a, 3.1b) are formed in one piece with the actuating element (3).

2. Tool according to claim 1, **characterized in that** the tool has at least one position securing device for axially fixing the at least one manipulator element (2).

3. Tool according to claim 1, **characterized in that** the at least one manipulator element (2) is designed as a scissor mechanism or as a spreader.

4. Tool according to one of claims 1 to 3, **characterized in that** the at least one actuating element (3) has at least one opening for an actuation on its exposed end face.

5. Tool according to one of claims 1 to 4, **characterized in that** the tool is designed as a robot end effector.

6. Tool according to one of claims 1 to 5, **characterized in that** the tool consists of metal or a ceramic material.

7. Tool according to one of claims 1 to 6 for use in micro-invasive examinations and operations.

8. Tool according to one of claims 1 to 7, **characterized in that** it is produced in a single process sequence by micro laser sintering.

9. Method for producing a tool for use as a micromanipulator according to claim 1, **characterized in that** the tool, with the gaps between the movable parts, is completely manufactured by additive manufacturing by means of micro laser sintering in one machining process, wherein starting from a longitudinal side of the tool, it is built layer by layer, wherein each layer is applied from a metallic or ceramic powder with a particle size smaller than 20 µm and then melted by laser and then solidifies, wherein the powder is not melted in areas of the gaps between the parts to be produced that are intended to be movable relative to one another, and **in that** during the micro laser sintering the frame (1), the manipulator element (2), and the actuating element (3) are built layer by layer, wherein the gaps between them amount to a maximum of 25 µm, and **in that** a first layer of a powder is applied to a building platform and the tool completely produced by micro laser sintering is subsequently separated from the building platform by wire electrical discharge machining or another separation process.

## Revendications

1. Outil destiné à être utilisé comme micromanipulateur pour des applications médicales consistant en interventions minimal-invasives (page 3 du descriptif, lignes 21-22), comportant plusieurs pièces composées
- d'un châssis (1) orienté le long d'un axe longitudinal (L), qui est conçu comme un corps creux ouvert aux deux bouts,
- d'au moins un élément manipulateur (2) disposé sur une première face d'extrémité (1.1) du châssis (1) et
- d'au moins un élément actionneur (3) disposé sur une deuxième face d'extrémité (1.2) opposée à la première face d'extrémité (1.1), qui est supporté de façon coulissante dans le châssis (1) et mobile dans le sens axial le long de l'axe longitudinal (L) et qui est en liaison active avec l'élément manipulateur,
dans lequel les pièces composées du châssis (1), de l'au moins un élément actionneur (3) et de l'au moins un élément manipulateur (2) sont reliées les unes aux autres par engagement positif de façon mobile et l'outil est entièrement fabriqué par addition de matière, y compris tous les interstices nécessaires entre les parties reliées entre elles de façon mobile, **caractérisé en ce que** l'au moins un élément manipulateur (2) comporte au moins deux mors de préhension (2.1a, 2.1b),
- un premier mors de préhension (2.1a) étant relié de façon mobile au châssis (1) et à l'au moins un élément actionneur (3) et un deuxième mors de préhension (2.1b) étant relié de façon rigide au châssis (1) ou
- les premier et deuxième mors de préhension (2.1a, 2.1b) étant reliés de façon mobile au châssis (1) et à l'au moins un élément actionneur (3) et des première et deuxième goupilles d'actionnement (3.1b et 3.1a) étant formées sur l'élément actionneur (3), le premier mors de préhension (2.1b) disposé de façon pivotante présentant dans la première articulation (G1) un premier trou oblong incurvé (2.2b) dans lequel se met en prise une première goupille de guidage (1.3b) disposée transversalement par rapport à l'axe longitudinal (L) sur le châssis (1) et orientée vers l'extérieur et le deuxième mors de préhension (2.1a) disposé de façon pivotante présentant dans la deuxième articulation (G2) un deuxième trou oblong incurvé (2.2a) dans lequel se met en prise une deuxième goupille de guidage (1.3a) disposée sur le châssis (1), les premier et deuxième mors de préhension (2.1a, 2.1b) reliés de façon mobile au châssis (1) et à l'au moins un élément actionneur (3) étant disposés avec possibilité de rotation autour d'un axe d'une première goupille de palier et d'une deuxième (1.4a, 1.4b) qui est disposé transversalement par rapport à l'axe longitudinal (L) du châssis (1), de sorte qu'un centre de rotation de la première articulation (G1) se trouve dans un axe de la première goupille de palier (1.4b) et qu'un centre de rotation de la deuxième articulation (G2) se trouve dans un axe de la deuxième goupille de palier (1.4a),
- le châssis (1), l'élément manipulateur (2), l'élément actionneur (3) et les première et deuxième goupilles d'actionnement (3.1a, 3.1b), les première et deuxième goupilles de palier (1.4a, 1.4b) et les première et deuxième goupilles de guidage (1.3a, 1.3b) se composant du même matériau, les première et deuxième goupilles de palier (1.4a, 1.4b) et les première et deuxième goupilles de guidage (1.3a, 1.3b) étant formées d'une pièce avec le châssis (1) et les première et deuxième goupilles d'actionnement (3.1a, 3.1b) étant formées d'une pièce avec l'élément actionneur (3).

2. Outil selon la revendication 1, **caractérisé en ce qu'**il comporte au moins une sécurité de position pour fixer l'au moins un élément manipulateur (2) dans le sens axial.

3. Outil selon la revendication 1, **caractérisé en ce que** l'au moins un élément manipulateur (2) est conçu comme un mécanisme de ciseaux ou comme un écarteur.

4. Outil selon l'une des revendications 1 à 3, **caractérisé en ce que** l'au moins un élément actionneur (3) présente, sur sa face d'extrémité libre, au moins une ouverture pour un actionnement.

5. Outil selon l'une des revendications 1 à 4, **caractérisé en ce qu'**il est conçu comme un effecteur terminal de robot.

6. Outil selon l'une des revendications 1 à 5, **caractérisé en ce qu'**il se compose de métal ou d'un matériau céramique.

7. Outil selon l'une des revendications 1 à 6 destiné à être utilisé au cours d'examens et d'opérations micro-invasifs.

8. Outil selon l'une des revendications 1 à 7, **caractérisé en ce qu'**il est fabriqué au cours d'une seule opération de procédé par micro-frittage par laser.

9. Procédé pour la fabrication d'un outil destiné à être utilisé comme micromanipulateur selon la revendication 1, **caractérisé en ce que** l'outil est entièrement fabriqué, avec les interstices entre les pièces mobiles, par addition de matière en une seule opération de traitement au moyen d'un micro-frittage par laser, l'outil étant construit couche par couche à partir d'un côté longitudinal de celui-ci, chaque couche d'une poudre métallique ou céramique d'une taille de particules inférieure à 20 µm étant appliquée puis fondue au moyen d'un laser et se solidifiant ensuite, la poudre n'étant pas fondue dans la région des interstices entre les pièces à produire qui doivent être mobiles les unes par rapport aux autres, et **en ce que**, pendant le micro-frittage par laser, le châssis (1), l'élément manipulateur (2) et l'élément actionneur (3) sont construits couche par couche, les interstices entre eux mesurant au maximum 25 µm, et **en ce qu'**une première couche d'une poudre est appliquée sur une plate-forme de fabrication et l'outil fini fabriqué par micro-frittage par laser est ensuite séparé de la plate-forme de fabrication par électroérosion par fil ou par un autre procédé de séparation.
